(19) Europäisches Patentamt
European Patent Office
Office européen des brevets

(11) **EP 4 506 330 A1**

(12) **EUROPEAN PATENT APPLICATION**
published in accordance with Art. 153(4) EPC

(43) Date of publication:
**12.02.2025 Bulletin 2025/07**

(21) Application number: **23827446.8**

(22) Date of filing: **15.06.2023**

(51) International Patent Classification (IPC):
*C07C 67/62* $^{(2006.01)}$    *C07C 69/94* $^{(2006.01)}$
*C08J 11/10* $^{(2006.01)}$    *C08G 63/06* $^{(2006.01)}$
*C08G 63/60* $^{(2006.01)}$    *C08G 63/85* $^{(2006.01)}$
*C08J 5/18* $^{(2006.01)}$

(52) Cooperative Patent Classification (CPC):
**C07C 67/62; C07C 69/94; C08G 63/06;**
**C08G 63/60; C08G 63/85; C08J 5/18; C08J 11/10**

(86) International application number:
**PCT/KR2023/008270**

(87) International publication number:
**WO 2023/249327 (28.12.2023 Gazette 2023/52)**

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**KH MA MD TN**

(30) Priority: **23.06.2022 KR 20220076777**

(71) Applicant: **SK Chemicals Co., Ltd.**
**Seongnam-si, Gyeonggi-do 13494 (KR)**

(72) Inventors:
• **KIM, Ha-Neul**
**Seongnam-si, Gyeonggi-do 13494 (KR)**
• **LEE, Yoo Jin**
**Seongnam-si, Gyeonggi-do 13494 (KR)**
• **HWANG, Da-Young**
**Seongnam-si, Gyeonggi-do 13494 (KR)**

(74) Representative: **Dehns**
**10 Old Bailey**
**London EC4M 7NG (GB)**

(54) **METHOD FOR STORING RECYCLED BIS(2-HYDROXYETHYL)TEREPHTHALATE AND METHOD FOR PREPARING POLYESTER RESIN**

(57)     Recycled bis(2-hydroxyethyl)terephthalate, obtained by depolymerization of a waste polyester, is mixed and stored with a solvent under specific conditions, leading to the stable maintenance of purity and yellowness and the improvement in product quality, including colors, of a final polyester resin prepared therefrom.

EP 4 506 330 A1

## Description

## Technical Field

The present invention relates to a method for storing recycled bis(2-hydroxyethyl) terephthalate and to a process for preparing a polyester resin. In addition, the present invention relates to a polyester resin prepared using the above process and to an article comprising the same.

## Background Art

Polyester is widely used as a material for beverage-filling containers, packaging films, audio and video films, and the like by virtue of its excellent mechanical strength, thermal resistance, transparency, and gas barrier properties. In addition, polyester is widely produced worldwide as an industrial material such as medical fibers and tire cords. In particular, polyester sheets or plates have good transparency and excellent mechanical strength, so that they are widely used as raw materials for cases, boxes, partitions, shelves, panels, packaging materials, building materials, interior and exterior materials, and the like.

As a result, waste of plastics such as polyester is generated globally at an unmanageable level every year. Recently, countries around the world are preparing regulations and plans for recycling waste plastic resources, including waste polyester. For example, there is an attempt to use a recycled resin in packaging materials used in various fields at a certain ratio or more. Although physical or chemical methods are used as methods of recycling waste polyester, physical recycling methods cannot guarantee purity and are not widely used.

In chemical recycling methods, the ester bond of waste polyester is cleaved to depolymerize it. Reactions such as glycolysis, hydrolysis, methanolysis, and aminolysis are used. Glycolysis among them is to decompose waste polyester by adding a glycol such as ethylene glycol or diethylene glycol at high temperatures. A reaction product containing mainly bis(2-hydroxyethyl) terephthalate (BHET) is obtained. The bis(2-hydroxyethyl) terephthalate may be used as a raw material for preparing unsaturated polyester or ester polyol after the crystallization or purification thereof.

In this regard, Korean Patent No. 1386683 discloses a crystallization method and apparatus for the chemical recycling of waste polyester. U.S. Patent No. 7,211,193 discloses a method for purifying bis(2-hydroxyethyl) terephthalate (BHET), which comprises subjecting a solution produced by the decomposition of a polyester containing polyethylene terephthalate (PET) as the main component using ethylene glycol (EG) to crystallization under a certain temperature condition and to solid-liquid separation.

[Prior Art Document]

(Patent Document 1) Korean Patent No. 1386683
(Patent Document 2) U.S. Patent No. 7211193

## Disclosure of Invention

## Technical Problem

Recycled bis(2-hydroxyethyl) terephthalate obtained through the depolymerization of waste polyester may be thereafter stored for a certain period of time before being put into the polymerization of a polyester resin. The quality of recycled BHET may deteriorate during the storage period, in which case the quality of a final polyester resin may be affected.

As a result of the study conducted by the present inventors, it has been discovered that, when recycled bis(2-hydroxyethyl) terephthalate is mixed with a solvent and stored under specific conditions, purity and yellow index can be stably maintained, and, accordingly, the quality such as the color of a final polyester resin produced therefrom can be enhanced.

Accordingly, an object of the present invention is to provide a method for stably storing a solution of recycled bis(2-hydroxyethyl) terephthalate and a process for preparing a polyester resin with high quality using the same.

## Solution to Problem

According to the present invention, there is provided a method for storing recycled bis(2-hydroxyethyl) terephthalate, which comprises (1) mixing recycled bis(2-hydroxyethyl) terephthalate obtained by the depolymerization of waste polyester with a solvent to prepare a solution of recycled bis(2-hydroxyethyl) terephthalate having a concentration of 95% by weight or less; and (2) storing the solution of recycled bis(2-hydroxyethyl) terephthalate at a temperature of 120°C or lower to maintain the YID index defined below to be 2 or less:

$$\text{YID index} = (\text{YID} - \text{YID [day 0]}) / \text{YID [day 0]}$$

Here, YID [day 0] is the yellow index of the solution of recycled bis(2-hydroxyethyl) terephthalate when 30 minutes has passed from the preparation thereof in step (1), and YID is the yellow index of the solution of recycled bis(2-hydroxyethyl) terephthalate after storage in step (2).

In addition, according to the present invention, there is provided a process for preparing a polyester resin, which comprises polymerizing a polyester resin using recycled bis(2-hydroxyethyl) terephthalate stored according to the above method.

In addition, according to the present invention, there is provided a polyester resin, which comprises recycled bis(2-hydroxyethyl) terephthalate stored according to the above method.

In addition, according to the present invention, there is provided an article, which comprises the polyester resin.

**Advantageous Effects of Invention**

According to the present invention, recycled bis(2-hydroxyethyl) terephthalate obtained by the depolymerization of waste polyester is mixed with a solvent and stored under specific temperature and concentration conditions to suppress a deterioration in purity and yellow index. Thus, the quality such as the color of a final polyester resin prepared therefrom can be enhanced.

Specifically, according to the present invention, since the change in purity and yellow index of a recycle BHET solution is very small even after storage for a certain period of time, even if it is put into a polymerization reaction after storage for a certain period of time, the color quality of a final polyester resin obtained therefrom may hardly deteriorate.

Accordingly, the recycled BHET stored according to the present invention and a polyester resin prepared therefrom can be used in the preparation of articles made of environmentally friendly materials in various fields.

**Best Mode for Carrying out the Invention**

Hereinafter, the present invention will be described in more detail.

In this specification, terms referring to the respective components are used to distinguish them from each other and are not intended to limit the scope of the embodiment. In addition, in the present specification, a singular expression is interpreted to cover a plural number as well unless otherwise specified in the context.

In the present specification, the terms first, second, and the like are used to describe various components. But the components should not be limited by the terms. The terms are used for the purpose of distinguishing one element from another.

In the present specification, the term "comprising" is intended to specify a particular characteristic, region, step, process, element, and/or component. It does not exclude the presence or addition of any other characteristic, region, step, process, element and/or component, unless specifically stated to the contrary.

**Recycled bis(2-hydroxyethyl) terephthalate**

Bis(2-hydroxyethyl) terephthalate is an ester of two ethylene glycols and one terephthalic acid. For example, it is a compound formed as an intermediate in the process of preparing polyester such as polyethylene terephthalate (PET) through the polymerization of ethylene glycol and terephthalic acid or its ester.

Bis(2-hydroxyethyl) terephthalate (BHET), which is used as a polymerization raw material for the polyester resin according to the present invention, is obtained from waste polyester having a repeat unit of ethylene glycol and terephthalic acid like polyethylene terephthalate (PET) or glycol-modified polyethylene terephthalate (PETG). For example, it may be obtained by well-known depolymerization methods such as glycolysis, hydrolysis, and methanolysis.

Bis(2-hydroxyethyl) terephthalate (BHET) obtained by the depolymerization of waste polyester as described above is referred to as "recycled bis(2-hydroxyethyl) terephthalate (recycled BHET)," or abbreviated as r-BHET or rBHET, which needs to be understood as distinct from a pure BHET compound.

Specifically, recycled BHET may contain reagents or solvents used in various chemical steps during the depolymerization of waste polyester or by-products formed by side reactions with them. Thus, BHET recycled by a common depolymerization process may contain organic and inorganic impurities in addition to BHET as the main component. For this reason, recycled BHET can also be viewed as a kind of composition comprising two or more components, i.e., a BHET composition. It may be used as a polymerization raw material for producing a polyester resin.

Impurities contained in the recycled BHET may comprise, for example, diethylene glycol derivatives and unreacted monomers. The total content of impurities contained in the recycled BHET may be 10% by weight or more, 15% by weight or more, or 20% by weight or more, and may be 40% by weight or less, 35% by weight or less, 30% by weight or less, or 25% by weight or less.

The purity of the recycled BHET may be measured using liquid chromatography or the like. Specifically, the purity of the recycled BHET may be calculated by measuring the fraction (%) of the peak area of BHET out of the total peak area in a spectrum obtained using high-performance liquid chromatography (HPLC).

For example, the purity of the recycled BHET may be 99.9% or less, 99% or less, 95% or less, 90% or less, or 85% or less, and may be 60% or more, 65% or more, 70% or more, 75% or more, or 80% or more. Specifically, the purity of the BHET put into the transesterification reaction of the present invention may be 60% to 99.9%, more specifically, 65% to 99% or 70% to 99%.

In addition, the recycled bis(2-hydroxyethyl) terephthalate may have a yellow index (YID) of 3.0 or less as measured with a spectrophotometer in a solution of 25% by weight. Specifically, the yellow index may be 2.5 or less, 2.0 or less, 1.5 or less, or 1.0 or less.

Although the bis(2-hydroxyethyl) terephthalate used in the present invention may be prepared by the conventional depolymerization methods, it may be prepared by carrying out a depolymerization reaction in multiple stages in order to obtain a higher purity by reducing impurities such as diethylene glycol esters. Specifically, the bis(2-hydroxyethyl) terephthalate can be obtained by carrying out a depolymerization reaction in multiple stages, while significantly lowering the temperature of the latter stage, followed by ion exchange and distillation of an unreacted glycol after the depolymerization reaction.

The process for preparing recycled bis(2-hydroxyethyl) terephthalate according to an embodiment may comprise (1a) subjecting waste polyester to depolymerization by a first glycolysis reaction at a temperature of 180°C to 200°C to obtain a first reactant; (1b) subjecting the first reactant to depolymerization by a second glycolysis reaction at a temperature of 150°C to 170°C to obtain a second reactant; (1c) subjecting the second reactant to ion exchanging through an ion-exchange resin to obtain a third reactant; (1d) removing an unreacted glycol from the third reactant through distillation at a temperature of 150°C or lower to obtain a fourth reactant; and (1e) subjecting the fourth reactant to distillation to obtain crude bis(2-hydroxyethyl) terephthalate.

According to a specific embodiment, first, waste polyester is pulverized to a size of 4 mm or less to be prepared, ethylene glycol is added thereto, which is then subjected to a first glycolysis reaction at a temperature of 180°C to 200°C in the presence of a zinc acetate catalyst for about 2 hours, and ethylene glycol is further added thereto, which is then subjected to a second glycolysis reaction at a temperature of 150°C to 170°C for about 2 hours. Thereafter, it is cooled to 120°C or lower using a reduced pressure flash, a small amount of a filter aid is added thereto, which is then filtered to separate insoluble foreign substances through solid-liquid separation, and it is passed through a column filled with an ion-exchange resin to carry out ion exchange. Then, an unreacted glycol is recovered at a temperature of 100°C to 130°C, purification is carried out by thin film evaporation at 190°C to 250°C, and, finally, an adsorption-crystallization step is carried out to obtain bis(2-hydroxyethyl) terephthalate with high purity and high quality.

According to the above process, a two-stage glycolysis reaction (i.e., a first glycolysis reaction and a second glycolysis reaction) is carried out. If the solvation is expedited in the first glycolysis reaction, the transesterification reaction of the waste polyester can be performed under the conditions of lower temperatures and short reaction time in the second glycolysis reaction. Thus, it is possible to significantly reduce the concentration of diethylene glycol (DEG) naturally formed at a common glycolysis reaction temperature and to significantly reduce the content of diethylene glycol esters in bis(2-hydroxyethyl) terephthalate finally prepared.

**Preparation and storage of a solution of recycled bis(2-hydroxyethyl) terephthalate**

Recycled bis(2-hydroxyethyl) terephthalate (BHET) may be stored for a period of time before being put into the polymerization of a polyester resin.

Since recycled BHET is obtained by the depolymerization of waste polyester, many side reactions may take place depending on the storage conditions, such as temperature, as compared with a high-purity BHET monomer newly synthesized. As a result, the quality of recycled BHET may deteriorate depending on the storage conditions before being put into the polymerization reaction, in which case the quality of a final polyester resin may be affected. If such recycled BHET with deteriorated quality is used, the quality of a final polyester resin may be impaired.

According to the present invention, as recycled BHET is mixed with a solvent to prepare a solution with a specific concentration range, which is then stored at a specific temperature range, the production of by-products that would affect the color quality of a final polyester resin can be suppressed to ensure storage stability. Thereafter, the polymerization reaction is carried out using the recycled BHET solution stored in the above manner, whereby a high-quality polyester resin can be finally obtained while securing process stability during the feeding thereof.

First, the recycled BHET is mixed with a solvent to prepare a solution of recycled BHET having a concentration of 95% by weight or less. Within the above concentration range, it is advantageous for maintaining the quality of the recycled BHET solution during the storage period and for enhancing the properties of a polyester resin prepared therefrom. For example, the concentration of the recycled BHET solution may be 90% by weight or less, 85% by weight or less, 80% by weight or less, 75% by weight or less, 70% by weight or less, 65% by weight or less, 60% by weight or less, or 55% by weight or less, and may be 5% by weight or more, 10% by weight or more, 15% by weight or more, 20% by weight or more, 25% by weight or more, 30% by weight or more, 35% by weight or more, 40% by weight or more, or 45% by weight or more. As a specific example, the concentration of the recycled BHET solution may be 5% by weight to 95% by weight, 5% by weight to 55% by weight, 45% by weight to 95% by weight, or 25% by weight to 95% by weight.

Examples of the solvent used for preparing the solution of recycled BHET include water, ethylene glycol, methanol, and ethanol. As a specific example, the solvent may comprise at least one of water and ethylene glycol. If the solvent comprises water and ethylene glycol at the same time, the mixing weight ratio of water and ethylene glycol may be, for example, 1:5 or 1:2.

The temperature (dissolution temperature) at which the recycled BHET is mixed with the solvent may be, for example, 60°C or higher, 65°C or higher, 70°C or higher, or 75°C or higher, and may be 197°C or lower, 180°C or lower, 165°C or lower, 140°C or lower, 120°C or lower, 100°C or lower, 90°C or lower, or 80°C or lower. According to a specific embodiment, the mixing temperature may be 60°C to 120°C. Within the above preferred temperature range, it is advantageous for maintaining the quality of the recycled BHET solution during the storage period and for enhancing the properties of a polyester resin prepared therefrom.

In the step of preparing the recycled BHET solution, further addition or evaporation of the solvent may be further carried out after the mixing to adjust the concentration to a desired concentration.

Thereafter, the solution of recycled bis(2-hydroxyethyl) terephthalate is stored at a temperature of 120°C or lower. Within the above temperature range, it is advantageous for maintaining the quality of the recycled BHET solution during the storage period and for enhancing the properties of a polyester resin prepared therefrom. For example, the storage temperature of the recycled BHET solution may be 120°C or lower, 115°C or lower, 110°C or lower, 105°C or lower, 100°C or lower, 95°C or lower, or 90°C or lower, and may be 20°C or higher, 25°C higher, 40°C higher, 60°C higher, 70°C higher, or 80°C higher. According to an embodiment, the storage temperature of the recycled BHET solution may be 60°C to 120°C.

As an example, the storage temperature of the recycled BHET solution may be the same as the temperature (dissolution temperature) for preparing the recycled BHET solution. As a specific example, the recycled BHET solution may be prepared at a temperature of 60°C to 120°C and stored while the temperature condition is maintained.

The storage pressure of the recycled BHET solution may be, for example, 5 kgf/cm$^2$ or less, 3 kgf/cm$^2$ or less, 2 kgf/cm$^2$ or less, 1.5 kgf/cm$^2$ or less, or 1 kgf/cm$^2$ or less, and may be 0.1 kgf/cm$^2$ or more, 0.3 kgf/cm$^2$ or more, 0.5 kgf/cm$^2$ or more, or 1 kgf/cm$^2$ or more. As a specific example, the storage pressure of the recycled BHET solution may be 2 kgf/cm$^2$ or less. Within the above preferred pressure range, it is advantageous for maintaining the quality of the recycled BHET solution during the storage period and for enhancing the properties of a polyester resin prepared therefrom.

The storage period of the recycled BHET solution may be, for example, 30 days or shorter, 20 days or shorter, 15 days or shorter, 7 days or shorter, or 5 days or shorter, and may be 1 hour or longer, 6 hours or longer, 12 hours or longer, or 1 day or longer, 2 days or longer, or 3 days or longer. As a specific example, the storage period of the recycled BHET solution may be 7 days or shorter. Within the above period range, it is advantageous for maintaining the quality of the recycled BHET solution during the storage period and for enhancing the properties of a polyester resin prepared therefrom.

According to the process of the present invention, the yellow index is maintained at a certain level during the storage period of the recycled BHET solution. According to an embodiment, the YID index defined as below is maintained at 2 or less when the recycled BHET solution is stored.

$$\text{YID index} = (\text{YID} - \text{YID [day 0]}) / \text{YID [day 0]}$$

Here, YID [day 0] is the yellow index of the solution of recycled bis(2-hydroxyethyl) terephthalate when 30 minutes has passed from the preparation thereof in step (1), and YID is the yellow index of the solution of recycled bis(2-hydroxyethyl) terephthalate after storage in step (2).

For example, the YID index may be 2 or less, 1.5 or less, 1 or less, 0.7 or less, 0.5 or less, or 0.3 or less. As a specific example, the YID index may be 0 to 2, 0 to 1.5, 0 to 1, or 0 to 0.5.

According to the present invention, even if a recycled BHET solution is put into a polymerization reaction after its preparation and storage for a certain period of time, the color quality of a final polyester resin obtained therefrom may hardly deteriorate. According to an embodiment, in the method for storing recycled bis(2-hydroxyethyl) terephthalate, the discoloration index according to the following equation is 3 or less.

$$\text{Discoloration index} = \text{Col}_{L\text{-}b} \text{ [day 0]} - \text{Col}_{L\text{-}b}$$

Here, $\text{Col}_{L\text{-}b}$ [day 0] is a value obtained by subtracting the b value from the L value in the Hunter Lab color space of a first polyester resin injection-molded product prepared by using the recycled bis(2-hydroxyethyl) terephthalate as it is without mixing it with a solvent and storing it. $\text{Col}_{L\text{-}b}$ is a value obtained by subtracting the b value from the L value in the Hunter Lab color space of a second polyester resin injection-molded product prepared by using the recycled bis(2-hydroxyethyl) terephthalate after it has been mixed with a solvent and stored in steps (1) and (2). The first and second polyester resin injection-molded products are prepared to a thickness of 6 mm under the same polymerization and injection molding conditions except that each bis(2-hydroxyethyl) terephthalate is used.

For example, the discoloration index may be 3 or less, 2.5 or less, 2 or less, 1.5 or less, 1 or less, 0.5 or less, or 0.3 or less. As a specific example, the discoloration index may be 0 to 3, 0 to 2, 0 to 1.5, or 0 to 1.

In addition, according to the present invention, the purity of recycled BHET is maintained at a certain level during the storage period of the recycled BHET solution. According to an embodiment, the purity of the recycled bis(2-hydroxyethyl) terephthalate used in step (1) may be 60% or more, and the change in purity according to the following equation may be 20% or less.

$$\text{Change in purity} = \text{rBHET purity [day 0]} - \text{rBHET purity}$$

Here, rBHET purity [day 0] is the purity of the recycled bis(2-hydroxyethyl) terephthalate before it is mixed with a solvent in step (1). rBHET purity is the purity of the recycled bis(2-hydroxyethyl) terephthalate obtained by evaporating the solvent in the solution after it is stored in step (2).

For example, the change in purity may be 20% or less, 15% or less, 10% or less, 5% or less, 3% or less, or 1% or less. Specifically, the change in purity may be 0 to 3, 0 to 2, 0 to 1.5, or 0 to 1.

**Process for preparing a polyester resin**

The solution of recycled bis(2-hydroxyethyl) terephthalate prepared and stored by the above method is used to polymerize a polyester resin.

That is, the process for preparing a polyester resin according to the present invention comprises polymerizing a polyester resin using recycled bis(2-hydroxyethyl) terephthalate (BHET) stored above.

As an example, the solution of recycled bis(2-hydroxyethyl) terephthalate, as it is, may be put into a polymerization reaction for a polyester, which may be advantageous in terms of uniform supply of a raw material and reaction efficiency. In particular, when it is put into a polymerization reaction as a solution, it is possible to continuously introduce the recycled BHET, thereby enabling a continuous process of a uniform polymerization reaction.

As another example, the solvent in the solution of recycled bis(2-hydroxyethyl) terephthalate may be evaporated to obtain solid-phase recycled bis(2-hydroxyethyl) terephthalate, which may then be put into the polymerization reaction for a polyester resin. In such a case, there is an advantage in that the existing process and equipment for introducing solid-phase recycled BHET can be used as it is by storing it as a solution for thermal stability and then evaporating the solvent and putting it into the polymerization reaction.

In the polymerization, an esterification reaction (first polymerization reaction step) and a polycondensation reaction (second polymerization reaction step) may be sequentially carried out.

The polyester resin according to the present invention may be prepared by further adding terephthalic acid or a derivative thereof and/or ethylene glycol in addition to the recycled bis(2-hydroxyethyl) terephthalate. In addition, the polyester resin may be prepared as a copolymer by further adding other diacids such as dicarboxylic acids and/or other glycols such as diols as comonomers.

For example, at least one monomer selected from the group consisting of (a) a dicarboxylic acid such as terephthalic acid or a derivative thereof, (b) ethylene glycol or diethylene glycol, and (c) another diol component as a comonomer may be further put into the esterification reaction.

The dicarboxylic acid may comprise at least one selected from terephthalic acid and isophthalic acid.

The diol component added as a comonomer may comprise at least one selected from the group consisting of cyclohexanedimethanol, cyclohexanedimethanol derivatives, and isosorbide. The cyclohexanedimethanol derivative may be 4-(hydroxymethyl)cyclohexylmethyl-4-(hydroxymethyl)cyclohexanecarboxylate or 4-(4-(hydroxymethyl)cyclohexylmethoxymethyl)cyclohexylmethanol.

In addition, the diol component added as a comonomer may further comprise 1,2-propanediol, 1,3-propanediol, 2-methyl-1,3-propanediol, 2-methylene-1,3-propanediol, 2-ethyl-1,3-propanediol, 2-isopropyl-1,3-propanediol, 2,2-dimethyl-1,3-propanediol, 1,4-butanediol, 2,3-butanediol, 3-methyl-1,5-pentanediol, 3-methyl-2,4-pentanediol, 1,6-hexanediol, 1,2-cyclohexanediol, and 1,4-cyclohexanediol.

The esterification reaction may be carried out in the presence of an esterification reaction catalyst. For example, a zinc-based compound may be used. Specific examples of the zinc-based catalyst include zinc acetate, zinc acetate hydrate, zinc chloride, zinc sulfate, zinc sulfide, zinc carbonate, zinc citrate, zinc gluconate, or mixtures thereof.

The esterification reaction may be carried out, for example, at a pressure of 0 kgf/cm² to 10.0 kgf/cm² and a temperature of 150°C to 300°C. The esterification reaction conditions may be appropriately adjusted according to the specific characteristics of the polyester to be produced, the ratio of each component, or process conditions. Specifically, the pressure in the esterification reaction may be 0 kg/cm² to 5.0 kg/cm², more specifically, 0.1 kg/cm² to 3.0 kg/cm². In addition, the temperature in the esterification reaction may be 200°C to 270°C, more specifically, 240°C to 260°C.

The esterification reaction may be carried out in a batch or continuous type. In addition, recycled BHET, a dicarboxylic acid component, and a diol component, which are raw materials, may be separately introduced into the reactor, or two or more raw materials may be introduced in a mixed state. They may be introduced in a solid, liquid, or slurry form. As an example, the dicarboxylic acid component, the diol component, and the recycled BHET may be added individually or in combination and may be mixed with the terephthalic acid oligomer prepared in advance. The terephthalic acid oligomer may be prepared by, for example, reacting terephthalic acid with a diol such as ethylene glycol, cyclohexanedimethanol, and isosorbide. As another example, they may be introduced in the form of a slurry in which a dicarboxylic acid component and recycled BHET are mixed with a diol component.

More specifically, a diol component such as isosorbide, which is solid at room temperature, may be dissolved in water or ethylene glycol and then mixed with a dicarboxylic acid component such as terephthalic acid to prepare a slurry. Alternatively, isosorbide may be melted at 60°C or higher, which is then mixed with a dicarboxylic acid component such as terephthalic acid and other diol components to prepare a slurry. In addition, water may be additionally added to the mixed slurry to help increase the fluidity of the slurry. In addition, in a continuous type, liquid raw materials (e.g., solution of recycled BHET) may be continuously fed to a reactor using a pump or the like. The hourly feeding amount of raw materials can be determined by dividing the total amount of raw materials to be fed by time to achieve the target production per day (e.g., 50 t/day).

The mixture of the solution of recycled bis(2-hydroxyethyl) terephthalate and other additive components is left in the esterification reactor for a certain period of time, for example, 1 hour to 24 hours or 4 hours to 10 hours, which is then transferred to a polycondensation reactor. The polycondensation reaction may produce a polyester resin having a relatively low molecular weight through melt polymerization. In addition, a polyester resin having a relatively high molecular weight may be produced through solid-state polymerization after the melt polymerization.

The temperature in the polycondensation reaction may be 150°C to 300°C, specifically, 200°C to 290°C, more specifically, 260°C to 280°C. In addition, the pressure in the polycondensation reaction may be 0.01 mmHg to 600 mmHg, specifically, 0.05 mmHg to 200 mmHg, more specifically, 0.1 mmHg to 100 mmHg. As the reduced pressure condition is adopted in the polycondensation reaction, glycol, which is a by-product of the polycondensation reaction, can be removed from the system. If the pressure in the polycondensation reaction exceeds the range of 0.01 mmHg to 400 mmHg, the removal of by-products may be insufficient. In addition, if the temperature in the polycondensation reaction is lower than 150°C, a glycol as a by-product of the reaction cannot be effectively removed from the system; thus, the intrinsic viscosity of a final reaction product is low, resulting in a decrease in the physical properties of the final polyester resin. If the temperature in the polycondensation reaction exceeds 300°C, the possibility of yellowing of a final polyester resin increases. In addition, the polycondensation reaction may be carried out for a necessary period of time, for example, an average residence time of 1 hour to 24 hours, until the intrinsic viscosity of a final reaction product reaches an appropriate level.

In addition, the polycondensation reaction may be carried out in the presence of a polycondensation catalyst. The polycondensation catalyst may be, for example, a titanium-based compound, a germanium-based compound, an antimony-based compound, an aluminum-based compound, a tin-based compound, or a mixture thereof. Examples of the titanium compound include tetraethyl titanate, acetyltripropyl titanate, tetrapropyl titanate, tetrabutyl titanate, 2-ethylhexyl titanate, octylene glycol titanate, lactate titanate, triethanolamine titanate, acetylacetonate titanate, ethylacetoacetic ester titanate, isostearyl titanate, titanium dioxide, and the like. Examples of the germanium-based compound include germanium dioxide, germanium tetrachloride, germanium ethylene glycol oxide, germanium acetate, or mixtures thereof. Preferably, germanium dioxide can be used. Both crystalline and amorphous germanium dioxide may be used, and glycol-soluble ones may also be used. The amount of the polycondensation catalyst used may be such that the amount of titanium element relative to the weight of the polyester resin is about 1 ppm to 100 ppm, more preferably, about 1 ppm to 50 ppm.

In addition to the polycondensation catalyst, a stabilizer, a colorant, a crystallizing agent, an antioxidant, a branching agent, or the like may be further used. The timing of adding these additives is not particularly limited, and they may be added at any time during the preparation step of the polyester resin.

As the stabilizer, phosphorus-based compounds such as phosphoric acid, trimethyl phosphate, triethyl phosphate, and triethyl phosphonoacetate may be generally used. The amount thereof added may be such that 10 ppm to 200 ppm relative to the weight of the polyester resin based on the amount of elements. In addition, common colorants such as cobalt acetate and cobalt propionate may be exemplified as the colorant added to enhance the color of the polyester resin. The amount thereof added may be such that 10 ppm to 200 ppm relative to the weight of the polyester resin based on the amount of cobalt element. If necessary, anthraquinone-based compounds, perinone-based compounds, azo-based compounds, methine-based compounds, or the like may be used as an organic colorant. Commercially available toners such as Polysynthren Blue RLS from Clarient or Solvaperm Red BB from Clarient may be used. The amount of the organic compound colorant added may be adjusted to 0 to 50 ppm based on the weight of the polyester resin. A crystal nucleating agent, an ultraviolet absorber, a polyolefin resin, a polyamide resin, and the like may be exemplified as the crystallizing agent. Hindered phenol-based antioxidants, phosphite-based antioxidants, thioether-based antioxidants, or mixtures thereof may be exemplified as the antioxidant. Conventional branching agents having three or more functional groups, for example, trimellitic anhydride, trimethylol propane, trimellitic acid, or mixtures thereof may be exemplified as the branching agent.

### Composition and characteristics of the polyester resin

The polyester resin of the present invention is a polyester resin regenerated through the chemical recycling of waste polyester.

The polyester resin according to an embodiment of the present invention comprises recycled bis(2-hydroxyethyl) terephthalate stored by the process described above.

Specifically, since the polyester resin of the present invention is polymerized using recycled BHET, it comprises a repeat unit derived from recycled BHET in the polymer chain.

The content of recycled BHET in the polyester resin of the present invention may be 1% by weight or more, 5% by weight or more, 10% by weight or more, 30% by weight or more, 50% by weight or more, 70% by weight or more, or 90% by weight or more. In addition, the content of recycled BHET may be 100% by weight or less, 99% by weight or less, 80% by weight or less, 60% by weight or less, 40% by weight or less, or 20% by weight or less.

As an example, the recycled bis(2-hydroxyethyl) terephthalate may be employed in an amount of 10% by weight to 99% by weight based on the weight of the polyester resin.

Meanwhile, since bis(2-hydroxyethyl) terephthalate has a structure in which two ethylene glycols and one terephthalic acid are bonded, the polyester resin of the present invention may essentially comprise a repeat unit derived from ethylene glycol and terephthalic acid.

As described above, the polyester resin of the present invention comprises a diacid component and a glycol component as monomers constituting the same. In addition, the polyester resin of the present invention may further comprise an additional diacid component and an additional glycol component for the polymerization of polyester.

In the polyester resin of the present invention, the diacid component may be a dicarboxylic acid or a derivative thereof, and the glycol component may be a diol.

In particular, the dicarboxylic acid comprises terephthalic acid, and the physical properties such as thermal resistance, chemical resistance, and weather resistance of a polyester resin can be enhanced by terephthalic acid. For example, terephthalic acid may be employed in an amount of 5% by mole to 100% by mole based on the number of moles of the entire dicarboxylic acid. In addition, the terephthalic acid component may be formed from a terephthalic acid alkyl ester such as dimethyl terephthalic acid.

In addition, the diol comprises ethylene glycol or diethylene glycol, and ethylene glycol or diethylene glycol may contribute to enhancing the transparency and impact resistance of a polyester resin. For example, ethylene glycol and/or diethylene glycol may be employed in an amount of 5% by mole to 100% by mole based on the number of moles of the entire diol.

According to an embodiment, the polyester resin of the present invention may be a copolymerized resin comprising two or more dicarboxylic acid components and/or two or more diol components.

Specifically, the dicarboxylic acid component may further comprise an aromatic dicarboxylic acid component, an aliphatic dicarboxylic acid component, or a mixture thereof, other than terephthalic acid. The dicarboxylic acid other than terephthalic acid may be employed in an amount of 1% by mole to 30% by mole based on the weight of the entire dicarboxylic acid components.

The aromatic dicarboxylic acid component may be an aromatic dicarboxylic acid having 8 to 20 carbon atoms, preferably, 8 to 14 carbon atoms, or a mixture thereof. Examples of the aromatic dicarboxylic acid include isophthalic acid, naphthalenedicarboxylic acids such as 2,6-naphthalenedicarboxylic acid, diphenyl dicarboxylic acid, 4,4'-stilbendicarboxylic acid, 2,5-furandicarboxylic acid, 2,5-thiophenedicarboxylic acid, and the like, but it is not limited thereto.

The aliphatic dicarboxylic acid component may be an aliphatic dicarboxylic acid having 4 to 20 carbon atoms, preferably, 4 to 12 carbon atoms, or a mixture thereof. Examples of the aliphatic dicarboxylic acid include linear, branched, or cyclic aliphatic dicarboxylic acid components such as cyclohexanedicarboxylic acids such as 1,4-cyclohexanedicarboxylic acid and 1,3-cyclohexanedicarboxylic acid, phthalic acid, sebacic acid, succinic acid, isodecylsuccinic acid, maleic acid, fumaric acid, adipic acid, glutaric acid, azelaic acid, and the like, but it is not limited thereto.

In addition, the diol component may further comprise a comonomer other than ethylene glycol or diethylene glycol. The comonomer may comprise, for example, at least one selected from the group consisting of cyclohexanedimethanol, cyclohexanedimethanol derivatives, and isosorbide.

The cyclohexanedimethanol (e.g., 1,2-cyclohexanedimethanol, 1,3-cyclohexanedimethanol, and 1,4-cyclohexanedimethanol) may contribute to enhancing the transparency and impact resistance of a polyester resin produced. For example, cyclohexanedimethanol may be employed in an amount of 5% by mole to 90% by mole based on the number of moles of the entire diol. The cyclohexanedimethanol derivative may be 4-(hydroxymethyl)cyclohexylmethyl-4-(hydroxymethyl)cyclohexanecarboxylate or 4-(4-(hydroxymethyl)cyclohexylmethoxymethyl)cyclohexylmethanol. The cyclohexanedimethanol derivatives may be employed in an amount of 0.1% by mole to 25% by mole based on the number of moles of the entire diol.

Isosorbide may enhance the processability of the final polyester resin. Although the transparency and impact resistance of a polyester resin are enhanced by the diol component of cyclohexanedimethanol and ethylene glycol, shear fluidization characteristics should be improved and the crystallization rate should be delayed for processability; however, it is difficult to achieve this effect with cyclohexanedimethanol and ethylene glycol alone. Thus, if isosorbide is employed as a diol component, the shear fluidization characteristics are improved and the crystallization rate is delayed while transparency and impact resistance are maintained, whereby it is possible to improve the processability of a polyester resin produced. Preferably, isosorbide may be employed in an amount of 0.1% by mole to 50% by mole based on the number of moles of the entire diol.

As a specific example, the polyester resin comprises a diacid component and a glycol component, wherein the diacid component may comprise at least one selected from the group consisting of terephthalic acid, isophthalic acid, dimethyl isophthalate, phthalic acid, dimethyl phthalate, phthalic anhydride, 1,6-naphthalenedicarboxylic acid, dimethyl 2,6-naphthalenedicarboxylate, diphenyl dicarboxylic acid, 1,4-cyclohexanedicarboxylic acid, 1,3-cyclohexanedicarboxylic acid, dimethyl 1,4-cyclohexanedicarboxylate, dimethyl 1,3-cyclohexanedicarboxylate, sebacic acid, succinic acid, isodecylsuccinic acid, maleic acid, maleic anhydride, fumaric acid, adipic acid, glutaric acid, and azelaic acid, and the glycol component may comprise at least one selected from the group consisting of isosorbide, ethylene glycol, 1,2-propanediol, 1,3-propanediol, 2-methyl-1,3-propanediol, 2-methylene-1,3-propanediol, 2-ethyl-1,3-propanediol, 2-isopropyl-1,3-propanediol, 2,2-dimethyl-1,3-propanediol, 1,4-butanediol, 2,3-butanediol, 3-methyl-1,5-pentanediol, 3-methyl-2,4-pentanediol, 1,6-hexanediol, 1,2-cyclohexanediol, 1,4-cyclohexanediol, diethylene glycol, 4-(hydroxymethyl)cyclohexylmethyl-4-(hydroxymethyl)cyclohexanecarboxylate, and 4-(4-(hydroxymethyl)cyclohexylmethoxymethyl)cyclohexylmethanol.

According to an embodiment, the polyester resin may further comprise at least one monomer selected from the group consisting of (a) a dicarboxylic acid or a derivative thereof, (b) ethylene glycol or diethylene glycol, and (c) a diol-based comonomer in addition to bis(2-hydroxyethyl) terephthalate. The dicarboxylic acid may comprise at least one selected from terephthalic acid and isophthalic acid. In addition, the diol-based comonomer may comprise at least one selected from the group consisting of cyclohexanedimethanol, cyclohexanedimethanol derivatives, and isosorbide. The cyclohexanedimethanol derivative may be 4-(hydroxymethyl)cyclohexylmethyl-4-(hydroxymethyl)cyclohexanecarboxylate or 4-(4-(hydroxymethyl)cyclohexylmethoxymethyl)cyclohexylmethanol. The diol-based comonomer may further comprise 1,2-propanediol, 1,3-propanediol, 2-methyl-1,3-propanediol, 2-methylene-1,3-propanediol, 2-ethyl-1,3-propanediol, 2-isopropyl-1,3-propanediol, 2,2-dimethyl-1,3-propanediol, 1,4-butanediol, 2,3-butanediol, 3-methyl-1,5-pentanediol, 3-methyl-2,4-pentanediol, 1,6-hexanediol, 1,2-cyclohexanediol, and 1,4-cyclohexanediol.

The polyester resin may also comprise a catalyst that has been used in the polymerization reaction for its preparation. For example, the polyester resin may comprise at least one catalyst selected from metal oxides and acetates. The metal contained in the catalyst may be selected from the group consisting of antimony (Sb), titanium (Ti), germanium (Ge), manganese (Mn), cobalt (Co), tin (Sn), and calcium (Ca).

The polyester resin may have a value obtained by subtracting the b value from the L value of 85 or more when Hunter Lab color space is measured. For example, the L - b value may be 85 or more, 86 or more, 87 or more, 88 or more, 89 or more, 90 or more, or 91 or more. In addition, the upper limit of the L - b value is not particularly limited. But it may be, for example, 100 or less, 99 or less, 98 or less, 97 or less, or 95 or less. The measurement of the Hunter Lab color space may be carried out by making a specimen having a thickness of 6 mm with the polyester resin.

The intrinsic viscosity of the polyester resin according to the present invention at 35°C may be 0.5 dl/g or more, 0.6 dl/g or more, or 0.7 dl/g or more, and may be 1.2 dl/g or less, 1.1 dl/g or less, 1.0 dl/g or less, or 0.9 dl/g or less. For example, the polyester resin may have an intrinsic viscosity of 0.5 dl/g to 1.2 dl/g at 35°C. Specifically, the polyester resin may have an intrinsic viscosity of 0.5 dl/g to 0.9 dl/g at 35°C.

The polyester resin according to the present invention can be used as a material for beverage-filling containers, packaging films, audio and video films, and the like by virtue of its excellent color, mechanical strength, thermal resistance, transparency, and gas barrier properties. In addition, polyester sheets or plates prepared from the polyester resin according to the present invention have good transparency and excellent mechanical strength, so that they can be used as raw materials for cases, boxes, partitions, shelves, panels, packaging materials, building materials, interior and exterior materials, and the like. In addition, the polyester resin according to the present invention can be used as an industrial material such as medical fibers and tire cords.

Accordingly, the present invention provides an article, which comprises the polyester resin. As an example, the article may be a film, a sheet, or a profile. Specific examples of the film include a heat shrinkable film and a blown film. The profile refers to a continuously extrusion-molded article of plastics excluding sheets and films. It may be manufactured by a general extrusion molding method and may have, for example, a tube or channel shape.

**Mode for the Invention**

Hereinafter, the present invention will be described in more detail with reference to embodiments. However, these examples are provided only for illustration purposes, and the present invention is not limited thereto.

**Preparation of recycled bis(2-hydroxyethyl) terephthalate**

Recycled BHET (rBHET) of various purities was prepared by the depolymerization of a waste polyester resin by a known method or purchased commercially. The table below shows the peak area fraction (%) of BHET, that is, purity, in the HPLC results of each recycled BHET (rBHET) measured using HPLC.

[Table 1]

| | rBHET #1 | rBHET #2 | rBHET #3 | rBHET #4 | rBHET #5 | rBHET #6 | rBHET #7 | rBHET #5-1 | rBHET #1-1 | rBHET #7-1 |
|---|---|---|---|---|---|---|---|---|---|---|
| Purity (HPLC - BHET%) | 97.0 | 97.0 | 95.5 | 95.5 | 90.0 | 80.0 | 84.6 | 90.0 | 79.0 | 96.0 |

### Example 1

### Step A: Preparation and storage of an r-BHET solution

rBHET #1 prepared above as recycled bis(2-hydroxyethyl) terephthalate was dissolved in ethylene glycol (EG) as a solvent at 120°C to obtain a solution having a concentration of 25% by weight. The solution of recycled bis(2-hydroxyethyl) terephthalate was stored at the same temperature (120°C) and a pressure of 1 kgf/cm$^2$ for 7 days and used as it was in the next step.

### Step B: Polymerization of a polyester resin

An esterification reactor was charged with the solution of recycled bis(2-hydroxyethyl) terephthalate obtained in the previous step (the weight of rBHET #1: 19,051.9 kg), terephthalic acid (TPA, 29,052.9 kg), ethylene glycol (EG, 6,665.7 kg), 1,4-cyclohexanedimethanol (CHDM, 4,320.4 kg), isosorbide (ISB, 1,460.1 kg), diethylene glycol (DEG, 265.0 kg), a Ge catalyst (64.0 kg), phosphoric acid (50.0 kg), a blue toner (0.050 kg), and a red toner (0.025 kg). Subsequently, nitrogen was injected into the esterification reactor to make the reactor pressurized by 2.0 kgf/cm$^2$ higher than normal pressure (absolute pressure: 2,231.1 mmHg). Then, the temperature of the esterification reactor was raised to 220°C over 90 minutes and maintained at 220°C for 2 hours, and the temperature was then raised again to 260°C over 2 hours. The mixture in the esterification reactor was stored at 260°C for about 7 hours and then transferred to a polycondensation reactor, and by-products formed during the reaction were discharged through a column and a condenser. Then, the pressure of the polycondensation reactor was reduced from normal pressure to 5 Torr (absolute pressure: 5 mmHg) over 30 minutes. At the same time, the temperature of the polycondensation reactor was raised to 280°C over 1 hour, and a polycondensation reaction was then carried out while the pressure of the polycondensation reactor was maintained at 1 Torr (absolute pressure: 1 mmHg) or less. At the beginning of the polycondensation reaction, the stirring speed may be set high. As the polycondensation reaction proceeds, when the stirring power is weakened due to the increase in the viscosity of the reactants or the temperature of the reactants rises above the set temperature, the stirring speed may be appropriately adjusted accordingly. The polycondensation reaction was carried out until the intrinsic viscosity (IV) of the mixture (melt) in the reactor reached 0.70 dl/g.

When the intrinsic viscosity of the mixture in the reactor reached the desired level, the mixture was then discharged to the outside of the reactor to form pellets, which were solidified with a cooling liquid and then granulated to have an average weight of about 12 to 14 mg. The granules were left at 150°C for 1 hour to be crystallized, which were then fed to a solid-state polymerization reactor. While nitrogen flowed at a rate of 50 L/minute, the temperature of the reactor was raised from room temperature to 190°C at a rate of 40°C/hour. While it was maintained, a solid-state polymerization was carried out until the intrinsic viscosity (IV) of the granules in the reactor reached 1.20 dl/g to obtain about 50 tons of a polyester resin (copolymer).

### Example 2

The same procedure as in Example 1 was repeated to obtain about 50 tons of a polyester resin (copolymer), except that, in step A, r-BHET #2 was used as recycled bis(2-hydroxyethyl)terephthalate, water was used as a solvent, the concentration was 25% by weight, the dissolution and storage temperature was 100°C, and the storage pressure was 2 kgf/cm$^2$; and that, in step B, the reactor was charged with the solution of recycled bis(2-hydroxyethyl) terephthalate (the weight of rBHET #2: 64,677.1 kg), ethylene glycol (EG, 263.1 kg), 1,4-cyclohexanedimethanol (CHDM, 1,833.4 kg), isosorbide (ISB, 495.7 kg), diethylene glycol (DEG, 539.8 kg), a Ge catalyst (32.0 kg), a blue toner (0.150 kg), and a red toner (0.075 kg), the polycondensation reaction was carried out until the intrinsic viscosity (IV) of the mixture (molten material) in the reactor reached 0.60 dl/g, and the solid-state polymerization was carried out until the intrinsic viscosity (IV) of the granules in the reactor reached 0.85 dl/g.

### Example 3

The same procedure as in Example 1 was repeated to obtain about 50 tons of a polyester resin (copolymer), except that, in step A, r-BHET #3 was used as recycled bis(2-hydroxyethyl)terephthalate, water was used as a solvent, the concentration was 70% by weight, the dissolution and storage temperature was 90°C, and the storage pressure was 1 kgf/cm$^2$; and that, in step B, the reactor was charged with the solution of recycled bis(2-hydroxyethyl) terephthalate (the weight of rBHET #3: 48,636.6 kg), terephthalic acid (TPA, 10,595.4 kg), ethylene glycol (EG, 474.9 kg), 1,4-cyclohexanedimethanol (CHDM, 735.3 kg), diethylene glycol (DEG, 1,353.1 kg), a Ge catalyst (32.0 kg), a Ti catalyst (4.5 kg), phosphoric acid (5.0 kg), a blue toner (0.200 kg), and a red toner (0.050 kg), the polycondensation reaction was carried out until the intrinsic viscosity (IV) of the mixture (molten material) in the reactor reached 0.70 dl/g, and the solid-state polymerization was not carried out.

**Example 4**

The same procedure as in Example 1 was repeated to obtain about 50 tons of a polyester resin (copolymer), except that, in step A, r-BHET #4 was used as recycled bis(2-hydroxyethyl)terephthalate, ethylene glycol (EG) was used as a solvent, the concentration was 90% by weight, the dissolution and storage temperature was 120°C, and the storage pressure was 1 kgf/cm$^2$; and that, in step B, the reactor was charged with the solution of recycled bis(2-hydroxyethyl) terephthalate (the weight of rBHET #4: 6,392.2 kg), terephthalic acid (TPA, 37,598.1 kg), ethylene glycol (EG, 13,106.5 kg), 1,4-cyclohexanedimethanol (CHDM, 2,174.4 kg), diethylene glycol (DEG, 1,333.8 kg), a Ti catalyst (2.2 kg), and phosphoric acid (10.0 kg), the polycondensation reaction was carried out until the intrinsic viscosity (IV) of the mixture (molten material) in the reactor reached 0.60 dl/g, and the solid-state polymerization was carried out until the intrinsic viscosity (IV) of the granules in the reactor reached 1.00 dl/g.

**Example 5**

The same procedure as in Example 1 was repeated to obtain about 50 tons of a polyester resin (copolymer), except that, in step A, r-BHET #5 was used as recycled bis(2-hydroxyethyl)terephthalate, water was used as a solvent, the concentration was 95% by weight, the dissolution and storage temperature was 100°C, and the storage pressure was 1.5 kgf/cm$^2$; and that, in step B, the reactor was charged with the solution of recycled bis(2-hydroxyethyl) terephthalate (the weight of rBHET #5: 49,166.5 kg), terephthalic acid (TPA, 5,670.4 kg), ethylene glycol (EG, 706.0 kg), 1,4-cyclohexanedimethanol (CHDM, 10,493.7 kg), a CHDM derivative (comprising 4-(hydroxymethyl)cyclohexylmethyl-4-(hydroxymethyl)cyclohexanecarboxylate and 4-(4-(hydroxymethyl)cyclohexylmethoxymethyl)cyclohexylmethanol at a molar ratio of 1:3, 997.4 kg), a Ge catalyst (64.0 kg), a Ti catalyst (4.5 kg), phosphoric acid (5.0 kg), cobalt acetate (6.3 kg), a blue toner (0.030 kg), and a red toner (0.010 kg), the polycondensation reaction was carried out until the intrinsic viscosity (IV) of the mixture (molten material) in the reactor reached 0.78 dl/g, and the solid-state polymerization was not carried out.

**Example 6**

The same procedure as in Example 1 was repeated to obtain about 50 tons of a polyester resin (copolymer), except that, in step A, r-BHET #6 was used as recycled bis(2-hydroxyethyl)terephthalate, ethylene glycol (EG) was used as a solvent, the concentration was 85% by weight, the dissolution and storage temperature was 120°C, and the storage pressure was 1 kgf/cm$^2$; and that, in step B, the reactor was charged with the solution of recycled bis(2-hydroxyethyl) terephthalate (the weight of rBHET #6: 13,403.4 kg), terephthalic acid (TPA, 26,279.1 kg), 1,4-cyclohexanedimethanol (CHDM, 19,756.8 kg), isosorbide (ISB, 6,163.5 kg), diethylene glycol (DEG, 2,237.4 kg), a Ge catalyst (320.2 kg), phosphoric acid (1.0 kg), a blue toner (0.150 kg), and a red toner (0.050 kg), the polycondensation reaction was carried out until the intrinsic viscosity (IV) of the mixture (molten material) in the reactor reached 0.70 dl/g, and the solid-state polymerization was not carried out.

**Example 7**

The same procedure as in Example 1 was repeated to obtain about 50 tons of a polyester resin (copolymer), except that, in step A, r-BHET #7 was used as recycled bis(2-hydroxyethyl)terephthalate, ethylene glycol (EG) was used as a solvent, the concentration was 50% by weight, the dissolution and storage temperature was 120°C, and the storage pressure was 1 kgf/cm$^2$; and that, in step B, the reactor was charged with the solution of recycled bis(2-hydroxyethyl) terephthalate (the weight of rBHET #7: 31,552.1 kg), terephthalic acid (TPA, 16,871.4 kg), isophthalic acid (IPA, 20,620.7 kg), ethylene glycol (EG, 2,660.6 kg), 1,4-cyclohexanedimethanol (CHDM, 10,082.2 kg), diethylene glycol (DEG, 2,394.1 kg), a Ti catalyst (0.9 kg), phosphoric acid (10.0 kg), and cobalt acetate (13.7 kg), the polycondensation reaction was carried out until the intrinsic viscosity (IV) of the mixture (molten material) in the reactor reached 0.82 dl/g, and the solid-state polymerization was not carried out.

**Comparative Example 1**

The same procedure as in Example 1 was repeated to obtain about 50 tons of a polyester resin (copolymer), except that, in step A, r-BHET #5-1 was used as recycled bis(2-hydroxyethyl)terephthalate, which was stored at 160°C and 1.5 kgf/cm$^2$ for 7 days without being mixed with a solvent; and that, in step B, the reactor was charged with the solution of recycled bis(2-hydroxyethyl) terephthalate (the weight of rBHET #5-1: 49,166.5 kg), terephthalic acid (TPA, 5,670.4 kg), ethylene glycol (EG, 706.0 kg), 1,4-cyclohexanedimethanol (CHDM, 10,493.7 kg), a CHDM derivative (comprising 4-(hydroxymethyl)cyclohexylmethyl-4-(hydroxymethyl)cyclohexanecarboxylate and 4-(4-(hydroxymethyl)cyclohexylmethoxymethyl)cyclohexylmethanol at a molar ratio of 1:3, 997.4 kg), a Ge catalyst (64.0 kg), a Ti catalyst (4.5 kg), phosphoric acid (5.0 kg), cobalt acetate (6.3 kg), a blue toner (0.030 kg), and a red toner (0.010 kg), the polycondensation reaction was carried out until the intrinsic viscosity (IV) of the mixture (molten material) in the reactor reached 0.78 dl/g, and the solid-state polymerization was not carried out.

**Comparative Example 2**

The same procedure as in Example 1 was repeated to obtain about 50 tons of a polyester resin (copolymer), except that, in step A, r-BHET #1-1 was used as recycled bis(2-hydroxyethyl)terephthalate, ethylene glycol (EG) was used as a solvent, the concentration was 98% by weight, the dissolution and storage temperature was 180°C, and the storage pressure was 1.8 kgf/cm$^2$; and that, in step B, the reactor was charged with the solution of recycled bis(2-hydroxyethyl) terephthalate (the weight of rBHET #1-1: 19,051.9 kg), terephthalic acid (TPA, 29,052.9 kg), ethylene glycol (EG, 6,665.7 kg), 1,4-cyclohexanedimethanol (CHDM, 4,320.4 kg), isosorbide (ISB, 1,460.1 kg), diethylene glycol (DEG, 265.0 kg), a Ge catalyst (64.0 kg), phosphoric acid (50.0 kg), a blue toner (0.050 kg), and a red toner (0.025 kg), the polycondensation reaction was carried out until the intrinsic viscosity (IV) of the mixture (molten material) in the reactor reached 0.70 dl/g, and the solid-state polymerization was carried out until the intrinsic viscosity (IV) of the granules in the reactor reached 1.20 dl/g.

**Comparative Example 3**

The same procedure as in Example 1 was repeated to obtain about 50 tons of a polyester resin (copolymer), except that, in step A, r-BHET #7-1 was used as recycled bis(2-hydroxyethyl)terephthalate, which was stored at 120°C and 1 kgf/cm$^2$ for 7 days without being mixed with a solvent; and that, in step B, the reactor was charged with the solution of recycled bis(2-hydroxyethyl) terephthalate (the weight of rBHET #7-1: 31,552.1 kg), terephthalic acid (TPA, 16,871.4 kg), ethylene glycol (EG, 2,660.6 kg), 1,4-cyclohexanedimethanol (CHDM, 10,082.2 kg), diethylene glycol (DEG, 2,394.1 kg), a Ti catalyst (0.9 kg), phosphoric acid (10.0 kg), and cobalt acetate (13.7 kg), the polycondensation reaction was carried out until the intrinsic viscosity (IV) of the mixture (molten material) in the reactor reached 0.78 dl/g, and the solid-state polymerization was not carried out.

**Test Example 1: Evaluation of the recycled BHET solution**

Evaluation of the recycled BHET solution was carried out as follows.

**(1) YID**

-    Measurement method: Transmittance data for the solution were obtained with Illuminant D65 using Color Flex EZ of Hunter Lab at an observer's angle of 2°. The yellow index (YID) value was calculated using a color analyzer in the software.
-    Measurement of YID [day 0]: Yellow index was measured 30 minutes after the recycled BHET solution was prepared (stored for 0 day) in step A of Examples 1 to 7 and Comparative Examples 1 to 3. In Comparative Examples 1 and 3, in which a solution was not prepared, the recycled BHET was dissolved in ethylene glycol (EG) at a concentration of 25% by weight at 120°C, and the yellow index was measured after 30 minutes.
-    Measurement of YID [day 1] and YID [day 7]: Yellow index was measured in the same manner as above after the recycled BHET solution was stored for 1 day and 7 days under the storage conditions in step A of Examples 1 to 7 and Comparative Examples 1 to 3.
-    The YID index was calculated according to the following equation.

$$\text{YID index} = (\text{YID [day 7]} - \text{YID [day 0]}) / \text{YID [day 0]}$$

Here, YID [day 0] is the yellow index of the recycled BHET solution when 30 minutes had passed from the preparation thereof, and YID [day 7] is the yellow index of the recycled BHET solution after storage for 7 days.

**(2) HPLC**

- Analysis method: About 0.01 g of a recycled BHET sample was diluted in about 20 ml of methanol, which was analyzed by high-performance liquid chromatography (HPLC) (model: Waters e2695, column: C18 (4.6 × 250 mm), 5 $\mu$m, UV detector: 242 nm, injection volume: 10 $\mu$l, eluent (gradient) A: $H_2O+H_3PO_4$, B: acetonitrile)
- Measurement of rBHET purity [day 0]: The recycled BHET used in step A of Examples 1 to 7 and Comparative Examples 1 to 3, respectively, was analyzed by HPLC before mixing with a solvent to determine the peak area fraction of BHET among the total peak areas.
- Measurement of rBHET purity [day 1], rBHET purity [day 3], rBHET purity [day 5], and rBHET purity [day 7]: Each recycled BHET solution was stored for 1, 3, 5, and 7 days under the storage conditions in step A of Examples 1 to 7 and Comparative Examples 1 to 3, the solvent was then evaporated, and it was analyzed by HPLC in the same manner as above. In Comparative Examples 1 and 3, in which the recycled BHET was not mixed with a solvent, the recycled BHET was stored in a solid state, and the yellow index was then measured.
- The change in purity was calculated according to the following equation.

$$\text{Change in purity} = \text{rBHET purity [day 0]} - \text{rBHET purity [day 7]}$$

Here, rBHET purity [day 0] is the purity of the recycled bis(2-hydroxyethyl) terephthalate before it is mixed with a solvent, and rBHET purity [day 7] is the purity of the recycled bis(2-hydroxyethyl) terephthalate obtained by evaporating the solvent in the solution after it was stored for 7 days.

Tables 2 and 3 below show the preparation and storage conditions and test results of the recycled BHET solutions in the Examples and Comparative Examples.

[Table 2]

| | | Unit | Ex. 1 | Ex. 2 | Ex. 3 | Ex. 4 | Ex. 5 |
|---|---|---|---|---|---|---|---|
| rBHET solution | rBHET | | rBHET #1 | rBHET #2 | rBHET #3 | rBHET #4 | rBHET #5 |
| | Solvent | | EG | Water | Water | EG | Water |
| | Concentration | % by weight | 25 | 25 | 70 | 90 | 95 |
| | Temperature | °C | 120 | 100 | 90 | 120 | 100 |
| | Pressure | kgf/cm$^2$ | 1 | 2 | 1 | 1 | 1.5 |
| YID | YID (day 0) | | 2.10 | 2.10 | 3.08 | 3.74 | 13.60 |
| | YID (day 1) | | 2.30 | 2.30 | 3.36 | 5.00 | 20.00 |
| | YID (day 7) | | 3.30 | 2.70 | 3.92 | 10.90 | 30.40 |
| | YID index | | 0.57 | 0.29 | 0.27 | 1.91 | 1.24 |
| Purity - HPLC | rBHET purity [day 0] | % | 97.0 | 97.0 | 95.5 | 95.5 | 90.0 |
| | rBHET purity [day 1] | % | 96.6 | 96.8 | 95.6 | 95.5 | 88.0 |
| | rBHET purity [day 3] | % | 96.4 | 96.4 | 95.0 | 95.0 | 87.6 |
| | rBHET purity [day 5] | % | 96.2 | 96.3 | 94.7 | 94.5 | 87.3 |
| | rBHET purity [day 7] | % | 96.2 | 96.3 | 94.6 | 94.3 | 87.0 |
| | Change in purity | % | 0.8 | 0.7 | 0.9 | 1.2 | 3.0 |

[Table 3]

| | | Unit | Ex. 6 | Ex. 7 | C. Ex. 1 | C. Ex. 2 | C. Ex. 3 |
|---|---|---|---|---|---|---|---|
| rBHET solution | rBHET | | rBHET #6 | rBHET #7 | rBHET #5-1 | rBHET #1-1 | rBHET #7-1 |
| | Solvent | | EG | EG | EG | EG | EG |
| | Concentration | % by weight | 85 | 50 | 100 | 98 | 100 |
| | Temperature | °C | 120 | 120 | 160 | 180 | 120 |
| | Pressure | kgf/cm$^2$ | 1 | 1 | 1 | 1.8 | 1 |
| YID | YID (day 0) | | 20.40 | 6.00 | 13.60 | 4.42 | 4.20 |
| | YID (day 1) | | 27.20 | 7.40 | 30.60 | 17.00 | 9.00 |
| | YID (day 7) | | 35.02 | 9.40 | 48.20 | 34.00 | 28.00 |
| | YID index | | 0.72 | 0.57 | 2.54 | 6.69 | 5.67 |
| Purity - HPLC | rBHET purity [day 0] | % | 80.0 | 84.6 | 90.0 | 79.0 | 96.0 |
| | rBHET purity [day 1] | % | 78.0 | 82.3 | 81.0 | 68.0 | 83.0 |
| | rBHET purity [day 3] | % | 75.0 | 81.5 | 77.5 | 65.0 | 76.0 |
| | rBHET purity [day 5] | % | 73.0 | 80.0 | 73.0 | 62.0 | 75.0 |
| | rBHET purity [day 7] | % | 72.0 | 79.0 | 65.0 | 60.0 | 73.0 |
| | Change in purity | % | 8.0 | 5.6 | 25.0 | 19.0 | 23.0 |

As can be seen from Tables 2 and 3 above, the recycled BHET solutions prepared and stored under the preferred concentration and temperature conditions in Examples 1 to 7 maintained high purity with a YID index of 2 or less after 7 days, indicating that a polyester resin with good quality would be produced therefrom.

In contrast, in Comparative Examples 1 to 3, the recycled BHET solution prepared and stored under conditions outside the preferred concentration and temperature showed a significant decrease in purity with a YID index exceeding 2 after 7 days, indicating that a polyester resin with poor quality would be produced therefrom.

**<Preparation and evaluation of a polyester resin>**

Preparation and evaluation of a polyester resin was carried out as follows.

**(0) Preparation of a polyester resin**

**<Test group>** Each polyester resin was prepared according to the procedures of Examples 1 to 7 and Comparative Examples 1 to 3 (that is, a recycled BHET solution was prepared and stored for 7 days and then subjected to a polymerization reaction to prepare a polyester resin).

**<Reference Group>** The procedures of Examples 1 to 7 and Comparative Examples 1 to 3 were carried out, except that the step of mixing the recycled BHET with a solvent or storing it under the specific temperature and pressure conditions (i.e., step A) was not carried out while step B was carried out to prepare a polyester resin.

**(1) Intrinsic viscosity (IV)**

A polyester resin was dissolved at a concentration of 0.12% in orthochlorophenol (OCP) at 150°C to obtain a solution, and an Ubbelohde viscometer was used in a constant temperature bath at 35°C to measure intrinsic viscosity. Specifically, the temperature of the viscous tube was maintained at 35°C, and the time (efflux time) required for the solvent to pass between specific internal sections of the viscous tube and the time required for the solution to pass to obtain specific viscosity, which was used to calculate intrinsic viscosity.

The intrinsic viscosity (i.e., melt IV) measured after the melt polymerization of a polyester resin and the intrinsic viscosity (i.e., solid-phase IV) measured after the solid-phase polymerization, following the melt polymerization, in Examples 1 to 7 and Comparative Examples 1 to 3 are shown in the tables below.

## (2) Composition

Each polyester resin was dissolved in a $CDCl_3$ solvent at a concentration of 3 mg/ml, whose $^1$H-NMR spectrum was obtained at 25°C using a nuclear magnetic resonance apparatus (JEOL, 600MHz FT-NMR). The content (% by mole) of diethylene glycol (DEG), isosorbide (ISB), and cyclohexanedimethanol (CHDM) residues based on the total number of moles of residues derived from all glycols (EG, DEG, BD, PDO, PTMG, PO3G, EO-PPG, and the like) was calculated by analyzing the above spectra, respectively.

The test for composition was carried out on the polyester resins of the Test Group (polymerization after a recycled BHET solution was prepared and stored for 7 days) and Reference Group (polymerization using recycled BHET as it was without mixing with a solvent or storing it).

## (3) Color

The chromaticity and brightness of samples were measured using a Varian Cary 5 UV/Vis/NIR spectrophotometer equipped with a diffuse reflection accessory. A polyester resin specimen with a thickness of 6 mm was prepared through injection molding at 250°C, for which transmittance data with Illuminant D65 at an observer's angle of 2° were obtained and processed using a color analyzer in Grams/32 software to calculate Hunter Lab values. The values of subtracting the b value from the L value (L - b) are calculated.

The test for color was carried out on the polyester resins of the Test Group (polymerization after a recycled BHET solution was prepared and stored for 7 days) and Reference Group (polymerization using recycled BHET as it was without mixing with a solvent or storing it).

The test results are shown in Tables 4 and 5 below.

[Table 4]

| | | Unit | Ex. 1 | Ex. 2 | Ex. 3 | Ex. 4 | Ex. 5 |
|---|---|---|---|---|---|---|---|
| Polyester resin | rBHET content | % by weight | 35 | 95 | 78 | 12 | 68 |
| | G/A | | 1.2 | 2.1 | 1.6 | 1.15 | 2.1 |
| Intrinsic viscosity | Melt IV | dl/g | 0.70 | 0.60 | 0.70 | 0.60 | 0.78 |
| | Solid-phase IV | dl/g | 1.20 | 0.85 | - | 1.00 | - |
| Composition - Reference Group (Day 0) | DEG content | % by mole | 0 | 2 | 5 | 4 | 3 |
| | ISB content | % by mole | 3 | 1 | 0 | 0 | 0 |
| | CHDM content | % by mole | 12 | 5 | 2 | 6 | 32 |
| Composition - Test Group (Day 7) | DEG content | % by mole | 0 | 2 | 5 | 4 | 3 |
| | ISB content | % by mole | 3 | 1 | 0 | 0 | 0 |
| | CHDM content | % by mole | 12 | 5 | 2 | 6 | 32 |
| Hunter Lab Color - Reference Group (Day 0) | L | | 93.30 | 92.70 | 92.00 | 92.30 | 92.30 |
| | b | | 0.20 | 0.15 | 0.40 | 0.10 | 0.25 |
| | L - b | (A) | 93.10 | 92.55 | 91.60 | 92.20 | 92.05 |
| Hunter Lab Color - Test Group (Day 7) | L | | 92.80 | 92.30 | 91.50 | 92.30 | 92.00 |
| | b | | 0.10 | 0.30 | 0.48 | 0.14 | 0.40 |
| | L - b | (B) | 92.70 | 92.00 | 91.02 | 92.16 | 91.60 |
| Discoloration index | (A) - (B) | | 0.40 | 0.55 | 0.58 | 0.04 | 0.45 |

[Table 5]

| | | Unit | Ex. 6 | Ex. 7 | C. Ex. 1 | C. Ex. 2 | C. Ex. 3 |
|---|---|---|---|---|---|---|---|
| Polyester resin | rBHET content | % by weight | 17 | 46 | 68 | 35 | 46 |
| | G/A | | 1.45 | 1.7 | 2.1 | 1.2 | 1.7 |
| Intrinsic viscosity | Melt IV | dl/g | 0.70 | 0.82 | 0.78 | 0.70 | 0.82 |
| | Solid-phase IV | dl/g | - | - | - | 1.20 | |
| Composition - Reference Group (Day 0) | DEG content | % by mole | 2 | 9 | 3 | 0 | 9 |
| | ISB content | % by mole | 15 | 0 | 0 | 3 | 0 |
| | CHDM content | % by mole | 65 | 31 | 32 | 12 | 31 |
| Composition - Test Group (Day 7) | DEG content | % by mole | 2 | 9 | 3 | 0 | 9 |
| | ISB content | % by mole | 15 | 0 | 0 | 3 | 0 |
| | CHDM content | % by mole | 65 | 31 | 32 | 12 | 31 |
| Hunter Lab Color - Reference Group (Day 0) | L | | 91.00 | 90.00 | 92.30 | 93.50 | 90.10 |
| | b | | 0.30 | 1.80 | 0.25 | 0.15 | 1.50 |
| | L - b | (A) | 90.70 | 88.20 | 92.05 | 93.35 | 88.60 |
| Hunter Lab Color - Test Group (Day 7) | L | | 90.00 | 89.80 | 90.00 | 91.50 | 88.30 |
| | b | | 0.70 | 2.00 | 1.40 | 1.70 | 4.00 |
| | L - b | (B) | 89.30 | 87.80 | 88.60 | 89.80 | 84.30 |
| Discoloration index | (A) - (B) | | 1.40 | 0.40 | 3.45 | 3.55 | 4.30 |

As can be seen from Tables 4 and 5 above, although the polyester resins of Examples 1 to 7 were prepared from recycled BHET stored for a certain period of time (7 days), there was no change in composition (DEG, ISB, and CHDM contents) as compared with the polyesters prepared from recycled BHET that had not been stored for a certain period of time (storage for 0 day). In particular, the discoloration index (change in Hunter L-b value) was very small, confirming that there was almost no deterioration in quality in terms of color.

In contrast, although the polyester resins of Comparative Examples 1 to 3 did not change in composition, the discoloration index (change in Hunter L-b value) exceeded 3, confirming that the color quality was remarkably deteriorated.

**Claims**

1. A method for storing recycled bis(2-hydroxyethyl) terephthalate, which comprises:

   (1) mixing recycled bis(2-hydroxyethyl) terephthalate obtained by the depolymerization of waste polyester with a solvent to prepare a solution of recycled bis(2-hydroxyethyl) terephthalate having a concentration of 95% by weight or less; and
   (2) storing the solution of recycled bis(2-hydroxyethyl) terephthalate at a temperature of 120°C or lower to maintain the YID index defined below to be 2 or less:

$$\text{YID index} = (\text{YID} - \text{YID [day 0]}) / \text{YID [day 0]}$$

   wherein YID [day 0] is the yellow index of the solution of recycled bis(2-hydroxyethyl) terephthalate when 30 minutes has passed from the preparation thereof in step (1), and YID is the yellow index of the solution of recycled bis(2-hydroxyethyl) terephthalate after storage in step (2).

2. The method for storing recycled bis(2-hydroxyethyl) terephthalate of claim 1, wherein the discoloration index according to the following equation is 3 or less:

$$\text{Discoloration index} = \text{Col}_{L\text{-}b} \, [\text{day } 0] - \text{Col}_{L\text{-}b}$$

wherein $\text{Col}_{L\text{-}b}$ [day 0] is a value obtained by subtracting the b value from the L value in the Hunter Lab color space of a first polyester resin injection-molded product prepared by using the recycled bis(2-hydroxyethyl) terephthalate as it is without mixing it with a solvent and storing it,

$\text{Col}_{L\text{-}b}$ is a value obtained by subtracting the b value from the L value in the Hunter Lab color space of a second polyester resin injection-molded product prepared by using the recycled bis(2-hydroxyethyl) terephthalate after it has been mixed with a solvent and stored in steps (1) and (2), and

the first and second polyester resin injection-molded products are prepared to a thickness of 6 mm under the same polymerization and injection molding conditions except that each bis(2-hydroxyethyl) terephthalate is used.

3. The method for storing recycled bis(2-hydroxyethyl) terephthalate of claim 1, wherein the purity of the recycled bis(2-hydroxyethyl) terephthalate used in step (1) is 60% or more, and the change in purity according to the following equation is 20% or less:

$$\text{Change in purity} = \text{rBHET purity} \, [\text{day } 0] - \text{rBHET purity}$$

wherein rBHET purity [day 0] is the purity of the recycled bis(2-hydroxyethyl) terephthalate before it is mixed with the solvent in step (1), and rBHET purity is the purity of the recycled bis(2-hydroxyethyl) terephthalate obtained by evaporating the solvent in the solution after it is stored in step (2).

4. The method for storing recycled bis(2-hydroxyethyl) terephthalate of claim 1, wherein the solvent comprises at least one of water and ethylene glycol.

5. The method for storing recycled bis(2-hydroxyethyl) terephthalate of claim 1, wherein the mixing temperature in step (1) is 60°C to 120°C.

6. The method for storing recycled bis(2-hydroxyethyl) terephthalate of claim 1, wherein the storage pressure in step (2) is 2 kgf/cm$^2$ or less.

7. The method for storing recycled bis(2-hydroxyethyl) terephthalate of claim 1, wherein the storage period in step (2) is 7 days or shorter.

8. A process for preparing a polyester resin, which comprises polymerizing a polyester resin using recycled bis(2-hydroxyethyl) terephthalate stored according to the method of claim 1.

9. A polyester resin, which comprises recycled bis(2-hydroxyethyl) terephthalate stored according to the method of claim 1.

10. The polyester resin of claim 9, wherein the content of the recycled bis(2-hydroxyethyl) terephthalate in the polyester resin is 10% by weight to 99% by weight.

11. The polyester resin of claim 9, wherein the polyester resin comprises at least one catalyst selected from metal oxides and acetates, and the metal is selected from the group consisting of antimony (Sb), titanium (Ti), germanium (Ge), manganese (Mn), cobalt (Co), tin (Sn), and calcium (Ca).

12. The polyester resin of claim 9, wherein the polyester resin comprises a diacid component and a glycol component,

the diacid component comprises at least one selected from the group consisting of terephthalic acid, isophthalic acid, dimethyl isophthalate, phthalic acid, dimethyl phthalate, phthalic anhydride, 1,6-naphthalenedicarboxylic acid, dimethyl 2,6-naphthalenedicarboxylate, diphenyl dicarboxylic acid, 1,4-cyclohexanedicarboxylic acid, 1,3-cyclohexanedicarboxylic acid, dimethyl 1,4-cyclohexanedicarboxylate, dimethyl 1,3-cyclohexanedicarboxylate, sebacic acid, succinic acid, isodecylsuccinic acid, maleic acid, maleic anhydride, fumaric acid, adipic acid, glutaric acid, and azelaic acid, and

the glycol component comprises at least one selected from the group consisting of isosorbide, ethylene glycol, 1,2-propanediol, 1,3-propanediol, 2-methyl-1,3-propanediol, 2-methylene-1,3-propanediol, 2-ethyl-1,3-propanediol, 2-isopropyl-1,3-propanediol, 2,2-dimethyl-1,3-propanediol, 1,4-butanediol, 2,3-butanediol, 3-methyl-1,5-pentanediol, 3-methyl-2,4-pentanediol, 1,6-hexanediol, 1,2-cyclohexanediol, 1,4-cyclohexanediol, diethylene glycol, 4-(hydroxymethyl)cyclohexylmethyl-4-(hydroxymethyl)cyclohexanecarboxylate, and 4-(4-(hydroxymethyl)cyclohexylmethoxymethyl)cyclohexylmethanol.

13. The polyester resin of claim 9, wherein the polyester resin has an intrinsic viscosity (IV) at 35°C of 0.5 dl/g to 1.2 dl/g.

14. An article, which comprises the polyester resin of any one of claims 9 to 13.

15. The article of claim 14, wherein the article is a film, a sheet, or a profile.

<table>
<tr><td colspan="2">**INTERNATIONAL SEARCH REPORT**</td><td>International application No.<br>**PCT/KR2023/008270**</td></tr>
</table>

| A. | CLASSIFICATION OF SUBJECT MATTER |
|---|---|

**C07C 67/62**(2006.01)i; **C07C 69/94**(2006.01)i; **C08J 11/10**(2006.01)i; **C08G 63/06**(2006.01)i; **C08G 63/60**(2006.01)i; **C08G 63/85**(2006.01)i; **C08J 5/18**(2006.01)i

According to International Patent Classification (IPC) or to both national classification and IPC

| B. | FIELDS SEARCHED |
|---|---|

Minimum documentation searched (classification system followed by classification symbols)

C07C 67/62(2006.01); C07C 67/29(2006.01); C07C 67/52(2006.01); C07C 67/54(2006.01); C07C 69/82(2006.01); C08G 63/78(2006.01); C08J 11/10(2006.01); C08J 11/24(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 비스(2-히드록시에틸)테레프탈레이트(bis(2-hydroxyethyl)terephthalate), 폴리에스테르(polyester), 해중합(depolymerization), 변색(discoloration)

| C. | DOCUMENTS CONSIDERED TO BE RELEVANT |
|---|---|

| Category* | Citation of document, with indication, where appropriate, of the relevant passages | Relevant to claim No. |
|---|---|---|
| A | KR 10-2022-0059681 A (SK CHEMICALS CO., LTD.) 10 May 2022 (2022-05-10)<br>See paragraphs [0004] and [0026]; and claims 9 and 13. | 1-15 |
| A | KR 10-2021-0066507 A (LOTTE CHEMICAL CORPORATION) 07 June 2021 (2021-06-07)<br>See entire document. | 1-15 |
| A | KR 10-2013-0120906 A (WOONGJIN CHEMICAL CO., LTD.) 05 November 2013 (2013-11-05)<br>See entire document. | 1-15 |
| A | WO 2021-032826 A1 (IONIQA TECHNOLOGIES B.V.) 25 February 2021 (2021-02-25)<br>See entire document. | 1-15 |
| A | KR 10-2002-0077874 A (AIES CO., LTD.) 14 October 2002 (2002-10-14)<br>See entire document. | 1-15 |

☐ Further documents are listed in the continuation of Box C.  ☑ See patent family annex.

| | |
|---|---|
| * Special categories of cited documents:<br>"A" document defining the general state of the art which is not considered to be of particular relevance<br>"D" document cited by the applicant in the international application<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| **12 September 2023** | **12 September 2023** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**
**Information on patent family members**

International application No.

**PCT/KR2023/008270**

| Patent document cited in search report | | | Publication date (day/month/year) | Patent family member(s) | | | Publication date (day/month/year) |
|---|---|---|---|---|---|---|---|
| KR | 10-2022-0059681 | A | 10 May 2022 | WO | 2022-097903 | A1 | 12 May 2022 |
| KR | 10-2021-0066507 | A | 07 June 2021 | | None | | |
| KR | 10-2013-0120906 | A | 05 November 2013 | KR | 10-1430108 | B1 | 18 August 2014 |
| WO | 2021-032826 | A1 | 25 February 2021 | CN | 114302903 | A | 08 April 2022 |
| | | | | EP | 4017839 | A1 | 29 June 2022 |
| | | | | EP | 4017839 | B1 | 07 June 2023 |
| | | | | JP | 2022-545677 | A | 28 October 2022 |
| | | | | KR | 10-2022-0050940 | A | 25 April 2022 |
| | | | | US | 2022-0267248 | A1 | 25 August 2022 |
| KR | 10-2002-0077874 | A | 14 October 2002 | CN | 1416415 | A | 07 May 2003 |
| | | | | EP | 1254888 | A1 | 06 November 2002 |
| | | | | EP | 1254888 | A4 | 14 April 2004 |
| | | | | JP | 4238300 | B2 | 18 March 2009 |
| | | | | KR | 10-0666883 | B1 | 10 January 2007 |
| | | | | US | 2003-0050499 | A1 | 13 March 2003 |
| | | | | US | 6710202 | B2 | 23 March 2004 |
| | | | | WO | 01-56970 | A1 | 09 August 2001 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1386683
- US 7211193 B